# EUROPEAN PATENT APPLICATION

(11) **EP 3 646 852 A1**
(43) Date of publication of application: **06.05.2020**
(21) Application number: 18203133.6
(22) Date of filing: 29.10.2018
(51) Int. Cl.: A61K 9/00, A61K 9/14, A61K 31/65, C07C 237/26, A61P 31/04

(54) **TETRACYCLINE COMPLEXES WITH SUSTAINED ACTIVITY**

(71) Applicant: Fraunhofer-Gesellschaft zur Förderung der angewandten Forschung e.V., 80686 München (DE); Martin-Luther-Universität Halle-Wittenberg, 06108 Halle (Saale) (DE)
(72) Inventor: KIESOW, Andreas, 06120 Halle (DE); BUCHHOLZ, Mirko, 06120 Halle (DE); SAREMBE, Sandra, 06120 Halle (DE); MÄDER, Karsten, 04105 Leipzig (DE); KIRCHBERG, Martin, 06114 Halle (DE); EICK, Sigrun, 3065 Bolligen (CH)
(74) Representative: Hoffmann Eitle

(57) **Abstract**

The present invention generally relates to controlled release of tetracycline antibiotics. Specifically, it relates to a complex comprising a tetracycline compound (TC) or a pharmaceutically acceptable salt, hydrate or solvate thereof and a divalent metal carboxylate; a pharmaceutical preparation comprising the complex, methods for manufacturing the complex and the pharmaceutical preparation, and a complex or a pharmaceutical preparation for use in a method for treatment of the human or animal body, in particular for therapy and/or prophylaxis of a bacterial infection; and/or wherein antibiotic activity is maintained over a prolonged period of time; and/or for the therapy and/or prophylaxis of an acute, chronic or recurrent periodontal disease.

## Description

### FIELD OF THE INVENTION

The present invention generally relates to controlled release of tetracycline antibiotics. Specifically, it relates to a complex comprising a tetracycline compound (TC) or a pharmaceutically acceptable salt, hydrate or solvate thereof and a divalent metal carboxylate; a pharmaceutical preparation comprising the complex, methods for manufacturing the complex and the pharmaceutical preparation, and a complex or a pharmaceutical preparation for use in a method for treatment of the human or animal body, in particular for therapy and/or prophylaxis of a bacterial infection; and/or wherein antibiotic activity is maintained over a prolonged period of time; and/or for the therapy and/or prophylaxis of an acute, chronic or recurrent periodontal disease.

### BACKGROUND ART

The tetracycline compounds are a class of antibiotics that inhibit protein synthesis by preventing the attachment of aminoacyl-tRNA to the ribosomal acceptor (A) site. The tetracyclines are broad-spectrum agents, exhibiting activity against a wide range of gram-positive and gram-negative bacteria, atypical organisms such as chlamydiae, mycoplasmas, and rickettsiae, and protozoan parasites. The favorable antimicrobial properties of these agents and the absence of major adverse side effects has led to their extensive use in the therapy of human and animal infections, for instance periodontal disease (Chopra et al., Microbiol Mol Biol Rev. 2001, June 65(2):232-60). Tetracycline molecules comprise a linear fused tetracyclic nucleus (rings designated A, B, C, and D) to which a variety of functional groups are attached. The simplest tetracycline to display detectable antibacterial activity is 6-deoxy-6-demethyltetracycline and so this structure may be regarded as the minimum pharmacophore (Mitscher, L. A. 1978. The chemistry of the tetracycline antibiotics. Marcel Dekker, Inc., New York, N.Y):

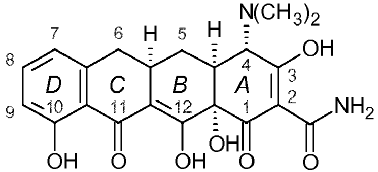

Tetracycline antibiotics that are widely clinically used include doxycycline, minocycline and tetracycline. An overview of the most commonly used tetracycline antibiotics is shown below:

| | | |
|---|---|---|
| 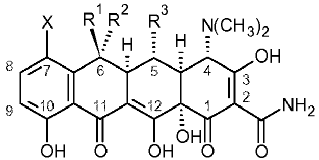 | **chlortetracycline:** | X = Cl, R¹ = OH, R² = Me, R³ = H |
| | **doxycycline:** | X = H, R¹ = H, R² = Me, R³ = OH |
| | **minocycline:** | X = N(CH₃)₂, R¹ = R² = R³ = H |
| | **oxytetracycline:** | X = H, R¹ = OH, R² = Me, R³ = OH |
| | **tetracycline:** | X = H, R¹ = OH, R² = Me, R³ = H |

Among these, especially doxycycline and minocycline have been used in the treatment of periodontal diseases. However, there are problems with the therapeutic applications of these active substances due to their relatively short retention time and their instability in aqueous environment.

Periodontal diseases are highly prevalent. About 30% of the human population is affected worldwide. They have considerable impact on individuals and society, and are costly to treat. The cost of dental care is the fourth highest of all diseases and consuming between 5 and 10% of all healthcare resources (Batchelor, P. British Dental Journal 2014, 217, 405-409). Representative population studies show that periodontal diseases are widespread and their prevalence has been increasing since 1997 (Micheelis, W. et al. Vierte Deutsche Mundgesundheitsstudie (DMS IV), Deutscher Arzte-Verlag, Köln, 2006). Amongst the adult population in Germany, 52.7% were found to be affected by moderately severe and 20.5% by severe forms of periodontitis. The health insurance expenditure in Germany for the direct treatment of periodontitis amounted to about EUR 1.1 billion (*Statistisches Bundesamt,* **2008**), not including the costs incurred by secondary diseases.

Periodontitis is a general term describing inflammation condition of the periodontal apparatus which is caused by multi-bacterial induction and has strong relations to various systemic diseases, such as cardiovascular diseases, rheumatoid arthritis, chronic obstructive pulmonary disease and Alzheimer's disease.

The currently established therapy of periodontitis, according to the recommendations of the German Society of Dental, Oral and Craniomandibular Sciences, is generally performed by manual supra and subgingival debridement (removal of the bacterial plaques) along with the application of antiseptic substances (daily disinfection by mouth washes), which disintegrates the entire oral biofilm and provides an opportunity for recolonization by potential pathogens. Furthermore, adjuvant systemic broad-spectrum antibiotic therapy is applied in advanced disease forms. The latter also leads to a non-selective destruction of the biofilm and has to be administered in high doses and over a prolonged period of time in order to reach sufficient therapeutic levels at the particular site of action, i.e. the gingival pocket. Standard adjuvant therapy of periodontitis involves, for instance, systemic administration of doxyciclin (per os) 1 × 200 mg/die for 1 day and 2 x 100 mg/die for further 18 days (Wissenschaftliche Stellungnahme: Adjuvante Antibiotika in der Parodontitistherapie, Deutsche Gesellschaft für Zahn- Mund- und Kieferheilkunde, *DZZ* **2003**). As a result, resistance development in oral pathogens is observed. Further, the microbiome in the patient's intestine is destroyed, which leads to a loss of metabolic support, immune modulation, and enables recolonization by potential pathogens. A focused and targeted therapy would represent a significant improvement in the treatment of periodontitis and conditions associated therewith.

The presence of periodontopathogenic bacteria varies among periodontitis patients. Nevertheless, the occurrence of certain bacterial species in the subgingival plaques has been found to be closely associated with the etiology of periodontal diseases (Socransky et al., Journal of Clinical Periodontology, 1998, 25, 134-144). The first step towards the development of periodontitis is the colonization of bacteria "yellow complex, "green complex" and "purple complex" in healthy periodontal sites. The *Actinomyces* species is closely related to members of the "yellow complex" *(Streptococcus sanguis, Streptococcus mitis, Streptococcus gordonii* and *Streptococcus intermedius),* "green complex" *(Eikenella corrodens, Capnocytophaga gingivalis, Capnocytophaga sputigena*), and "purple complex" (*Veillonella parvula, Actinomyces odontolyticus).* These complexes are followed by colonization by the members of the bacteria of the so-called "orange complex" including members of the *Fusobacterium nucleatum*/*periodonticum* subspecies, *Prevotella intermedia, Prevotella nigrescens* and *Parvimonas micra* (formerly known as *Peptostreptococcus micros* or *Micromonas micros*)*.* Colonization of healthy periodontal sites by members of the "orange complex" has been found to correlate with the occurrence of gingivitis. The bacteria of the "orange complex" furthermore promote the colonization by bacteria of the so-called "red complex", which in turn are associated with deep pockets and chronic periodontitis. The "red complex" consisting of the tightly related group *Tannerella forsythia, Porphyromonas gingivalis* and *Treponema denticola* strongly relates to clinical measures of periodontal disease and in particular to pocket depth and bleeding on probing. Additionally, Lamont et al. (Microbiology 2002, 148, 1627-1636.) and Daep et al. (Infect. Immun. 2006, 74, 5756-5762) demonstrated that S. *gordonii* plays a role in colonization with *Porphyromonas gingivalis.*

The application of antibiotics or antiseptics within an adhesive slow-release form of administration for topical application is technologically challenging. The oral mucosa is covered by a liquid film and is partially colonized by bacteria. Additionally, it is subject to mechanical stress due to speaking, swallowing and chewing. Although these stress sources are not present in the periodontal pocket per se, the presence of sulcus liquid (the flow rate of which is strongly increased in the case of periodontitis) leads to increased washing out of active substances out of the pocket.

In view of this difficult starting situation, none of the existing products or local antibiotic or antiseptic therapy has been able to solve the existing problems in a satisfactory manner. For instance, Actisite® is a tetracycline containing fiber for periodontal pocket placement requiring a complicated application by a specialized dentist into the periodontal pocket and a further intervention for removal after 10 days. This product is not on the German market anymore. Elyzol®, a metronidazol containing gel, fails to meet the expectations for active ingredient release beyond two days due to lack of sufficient gel adhesion. This product is also not on the German market anymore. Atridox®, a doxycycline containing controlled-release product composed of a two syringe mixing system, has also been taken off the German market.

Ligosan®, which is a doxycycline containing application system, and Periochip®, which is a chlorhexidine containing bio degradable chip, are the only products for local application still available on the market. Ligosan® is an application system comprising 14% of doxycycline in a biodegradable gel matrix for application in the periodontal pocket by means of a special location system, which provides a continuous release over 12 days and leads to an improvement of the periodontal pockets. However, the handling and manageability of the gel is complicated and application of the proper amount in all parts of the pocket is difficult, especially when not directly visible interdental spaces in the area of the side teeth are concerned. The gel needs to be inserted by means of a special application system. Furthermore, although it is proven to be effective against bacteria in the deep periodontal pockets, it is not suitable for a complete therapy and does not substitute classical treatment measures (Eickholz, P et al.; J Clin Periodontol 2002; 108-117; Ratka-Krüger, P. et al., J Periodontol. 2005/1,76: 66-74). Additionally, due to the high costs and low incentive for the dentists, a general application of this therapy appears to be difficult (see https://www.parodontitis.com/ligosan-slow-releaseR-zur-behandlung-von-zahnfleischtaschen.html). Periochip® contains 2.5mg of chlorhexidine in a biodegradable gelatin chip that can be inserted into the periodontal pockets with tweezers. The chip remains in the pocket for 7-10 days and over time, the drug is released continuously. However, it was found to be not as effective against the bacterial composition as local antibiotics. Further, the costs are not paid by the health insurance (see https://www.parodontitis.com/lokale-behandlung-der-zahnfleischtaschen-mit-periochipec40-chxhtml.html). In summary, although some improvement of periodontitis parameters (depth of the pocket and inflammation parameters) has been achieved with these systems, they require complicated application and furthermore are not reimbursed by the public health insurance companies in Germany such that they cause unbearable costs which have to be paid by the patients.

The systemic application of low-dose doxycycline (Periostat®) as a long-term therapy has also been applied. However, the expected pharmacologic effect does not always correlate to a successful clinical result. For instance, therapy seems to have diminished efficacy in smokers. Furthermore, a high level of compliance on the side of the patient is required; and the results of scientific studies as to whether a clinical improvement is present are controversial. Thus, no general recommendation can be given concerning this therapy (G. Greenstein, Efficacy of submicrobial-dose doxycycline in the treatment of periodontal diseases: a critical evaluation, The international journal of Periodontics & Restorative Dentistry, 2004, 24(6):528-543; I. Needleman et al., A randomized-controlled trial of low-dose doxycycline for periodontitis in smokers, Journal of clinical Periodontology, 2007, 34(4):325-333).

Finally, Arestin® is a subgingival sustained-release product composed of microspheres containing the antibiotic minocycline hydrochloride incorporated into a bioresorbable polymer, poly(glycolide-co-DL-lactide) or PGLA, for professional subgingival administration into periodontal pockets. Each unit-dose cartridge delivers minocycline hydrochloride equivalent to 1 mg of minocycline free base. The continuous drug release is described to be 14 days. Nevertheless, the product is no longer available on the German market (see https://www.parodontitis.com/vommarkt-genommen-atridoxR-actisiteR-arestinR-und-elyzolR.html). The consistency of the material (microspheres) requires application by using special cartridges and renders its handling and placement into the periodontal pocket difficult and not very reliable.

Thus, there is a high demand for the development of novel, improved forms for administration of tetracycline antibiotics with sustained activity, in particular for the treatment of periodontitis and related conditions.

### PROBLEMS TO BE SOLVED BY THE INVENTION

In view of the above, the present invention aims at providing a form for administration of tetracycline compounds having increased stability; and/or prolonged activity; and/or simpler, easier and/or more reliable applicability and handling. In particular, the present invention aims at providing topical application forms allowing a dentist to apply an antibiotic in a simple, reliable and as painless as possible manner. Further, significantly prolonged and uniform release is aimed at, such that the required activity level is maintained over a prolonged period of time (i.e. beyond 14 days). Finally, it would be desirable that the new forms for administration are preferably safe, reliable and sufficiently cheap to be reimbursed by the public health insurance.

Further objects of the present invention are the provision of a method for manufacturing such administration forms; the provision of a method for treatment of the human or animal body, and/or a compound or a pharmaceutical preparation for use in such method; the provision of a method for therapy or prophylaxis of a bacterial infection, and/or a compound or a pharmaceutical preparation for use in such method; the provision of a method for therapy or prophylaxis of an acute, chronic or recurrent periodontal disease and/or a compound or a pharmaceutical preparation compound for use in in such method.

### SUMMARY OF THE INVENTION

The present invention provides a complex comprising a tetracycline compound (TC) or a pharmaceutically acceptable salt thereof and a divalent metal carboxylate (MA₂), wherein: the molar ratio TC : MA₂ is in the range of 1 : 0.8-3.0; the divalent metal cation M is an earth alkaline metal cation; and A is a carboxylate anion derived from a C₈-C₂₄ carboxylic acid.

The present invention further provides a pharmaceutical preparation comprising the complex according to any one of aspects 1-4 and optionally one or more pharmaceutically acceptable excipient(s), preferably in the form of a strand-shaped extrudate

The present invention further provides a method for manufacturing said complex, the method comprising the following steps: (a) mixing a tetracycline compound (TC) or a pharmaceutically acceptable salt thereof and a divalent metal carboxylate (MA₂) in a molar ratio of 1 : 0.8-3.0 with an organic solvent to obtain a dispersion, wherein the divalent metal cation M is an earth alkaline metal cation and A is a carboxylate anion derived from a C₈-C₂₄ carboxylic acid; (b) heating said dispersion to form the complex; (c) removing the solvent to obtain the complex. The present invention further provides a method for manufacturing the pharmaceutical preparation in the form of a strand-shaped extrudate, the method comprising the following steps: (d) comminuting said complex and, if present, one or more pharmaceutically acceptable excipients to obtain an extrusion precursor; (e) extruding said extrusion precursor at a temperature above room temperature; (f) cooling the product of step (e) to obtain the pharmaceutical preparation in the form of a strand-shaped extrudate.

The present invention further provides a complex as defined above and/or a pharmaceutical preparation as defined above for use in a method for treatment of the human or animal body; and/or for use in a method for therapy and/or prophylaxis of a bacterial infection, preferably caused by one or more bacteria selected from the group consisting of *Porphyromonas gingivalis, Prevotella intermedia, Tannerella forsythia, Streptococcus gordonii, Fusobacterium nucleatum, Actinomyces naeslundii* and *Parvimonas micra;* and/or wherein antibiotic activity is maintained over a period of at least 21 days; and/or for use in a method for therapy and/or prophylaxis of an acute, chronic or recurrent periodontal disease.

### EFFECTS ACHIEVED BY THE INVENTION

The present inventors found that the complexes of tetracycline compounds according to the present invention degrade more slowly in aqueous environments than the corresponding tetracycline compounds. Furthermore, these complexes were found to have a lower solubility in water and to provide a more delayed and sustained release as compared to the corresponding tetracycline compounds or even to sustained release systems, such as Arestin®. Although chelate complexes of tetracycline compounds with magnesium or calcium ions in a ratio of 1:0.5 have been previously described, the present complexes are different in term of their stoichiometry. The complexation according to the present invention results in modified chemical properties of the compounds. In particular, the solubility, the photometric behavior and the reactivity are influenced in a favorable manner. The active substance remains biologically active over a prolonged period of time and is released in a retarded manner due to the lower solubility in water, increased stability towards degradation in aqueous solution and at the lipophilic nature of the carboxylate anion. Furthermore, the complex formation seems to prevent a conversion of the tetracycline compounds into less active epimers and thus contributes to maintaining the biological activity of the active substances over a longer period of time. These effects can be maintained when the complexes according to the present invention are administered either alone or in combination with one or more pharmaceutical excipients in a pharmaceutical preparation, e.g., a preparation for topical administration.

Moreover, when the complex is provided in a pharmaceutical preparation such as an extrudate (either as a pure substance or together with an excipient, e.g., a biodegradable polymer), an even longer release and activity can be achieved. Such extrudates can be easily formed and handled (e.g., in order to be brought into a periodontal pocket) and additionally result in a yet further delay of drug release. Finally, they exhibit good adhesion to the oral mucosa and thus excellent application reliability.

The manufacture method described herein leads to the formation of novel tetracycline compound complexes with surprisingly pronounced sustained drug release that is therapeutically desirable. Furthermore, the complexes obtained according to the manufacture method of the present invention have an increased solubility in organic solvents and decreased water solubility. The methods for manufacturing the complexes and preparations according to present invention are simple, versatile, do not require the use of toxic organic solvents, and can be based exclusively on using well established, reliable and safe pharmaceutical ingredients. Furthermore, the complexes according to the present invention are easily formable and can be processed by extrusion, either alone or together with one or more excipient in order to be converted into extrudate having a suitable desired geometry, e.g., suitable for easier placement into a periodontal pocket.

The complexes and preparations were shown to be superior in terms of release behavior and sustained antibacterial activity, especially against oral pathogens associated with the occurrence and progress of periodontitis. This can contribute to an increased efficacy of non-surgical periodontitis therapy and consequently to a decreased amount of surgical treatment necessary, along with a significant cost reduction. Additionally, the burden for the patient caused by treatment with systemically administered antibiotics can be reduced by the substitution for an effective locally applied therapeutic form instead.

### BRIEF DESCRIPTION OF THE DRAWINGS

**Fig. 1** shows glass vials with dried minocycline/magnesium stearate complexes in different molar ratios. From left to right: minocycline hydrochloride/magnesium stearate in molar ratios of 1:1, 1:2; minocycline free base/magnesium stearate in ratios of 1:1 and 1:2 (corresponding to Examples 4, 3, 2 and 1, respectively).
**Fig. 2** shows microphotographs of dried minocycline/magnesium stearate having a molar ratio of minocycline/magnesium stearate of 1:1 (left, Example 2) and 1:2 (right, Example 1).
**Fig. 3** shows microphotographs of dried doxycycline-magnesium stearate having a molar ratio of doxycycline/magnesium stearate of 1:1 (left, Example 6) and 1:2 (right, Example 5).
**Fig. 4** shows chromatograms of minocycline (left) and the complex according to Example 1 (right) during incubation in a buffer at pH 7.1 (top) and 2.3 (bottom) at 37°C over time (day zero always being the top curve).
**Fig. 5** shows UV/Vis absorption spectra of minocycline and the minocycline complex according to Example 1 in ethanol before (left) and after (right) addition of hydrochloric acid.
**Fig. 6** shows FTIR-ATR spectra of minocycline, magnesium stearate and the complex according to Example 1 in the area 680-4000 cm⁻¹.
**Fig. 7** shows release curves for the extrudate of Example 9 consisting of the pure minocycline/magnesium stearate complex 1:2, as well as together with various PLGA polymers upon extrusion (Examples 10-13).
**Fig. 8** shows force-displacement diagrams upon deformation of extrudates on a texture analyzer fitted with a knife edge blade.
**Fig. 9** shows a photograph of extrudates obtained according to the present invention comprising Resomer 502 (top) and Resomer 503 (bottom).
**Fig. 10** shows an experimental assembly for assessment of the adhesive force towards mucous membranes (A) and the geometry of the flow channel specifically used for the measurements (B).
**Fig. 11** shows the minimal inhibitory concentrations of eluates (dilutions) vs. *Streptococcus gordonii ATCC 10558* and *Porphyromonas gingivalis ATCC* 33277 up to 42 days.
**Fig. 12** shows the inhibitory effect of eluates obtained after 24 hours, 2 days, 7 days, 14 days and 28 days vs. biofilm formation.

### DETAILED DESCRIPTION OF THE INVENTION

### Complexes

The present invention provides a complex comprising a tetracycline compound (TC) or a pharmaceutically acceptable salt, hydrate or solvate thereof and a divalent metal carboxylate (MA₂), wherein: the molar ratio TC : MA₂ is in the range of 1 : 0.8-3.0; the divalent metal cation M is an earth alkaline metal cation; and A is a carboxylate anion derived from a C₈-C₂₄ carboxylic acid.

### Tetracycline compounds

As used herein, the term "tetracycline compound" (TC) refers to any compound belonging to the family of tetracycline antibiotics, i.e. based on the 6-deoxy-6-demethyltetracycline minimum pharmacophore structure:

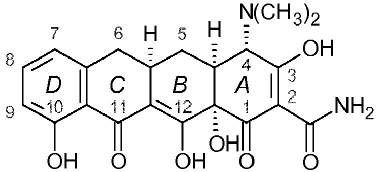

Preferably, the tetracycline compound (TC) is selected from minocycline, doxycycline, tetracycline, chlortetracycline, oxytetracycline, rolitetracycline, tigecycline, demeclocycline, lymecycline, meclocycline, methacycline, omadacycline, sarecycline and eravacycline. Preferred tetracycline compounds are also selected from the group consisting of 7-chlortetracycline, 5-hydroxytetracycline, tetracycline, 6-demethyl-7-chlortetracycline, 2-N-pyrrolidinomethyltetracycline, 2-N-lysinomethyltetracycline, N-methylol-7-chlortetracycline, 6-methylene-5-hydroxytetracycline (methacycline), 6-deoxy-5-hydroxytetracycline (doxycycline), 7-dimethylamino-6-demethyl-6-deoxytetracycline (minocycline), 9-(N,N-dimethylglycylamido)-6-demethyl-6-deoxytetracycline, 9-(N,N-dimethylglycylamido)-minocycline, 9-(t-butylglycylamido)-minocycline (tigecycline), as further described in Table 2 of Chopra et al., Microbiol Mol Biol Rev. 2001, June 65(2):232-60). Further preferred tetracycline compounds include clomocycline (N-methylol-7-chlortetracycline), 9-(N,N-dimethylglycylamido)-6-demethyl-6-deoxytetracycline and 9-(N,N-dimethylglycylamido)-minocycline.

It should be noted that the term "tetracycline compound" or "TC" as used herein denotes the general class of compounds, namely any compound belonging to the family of tetracycline antibiotics, whereas the term "tetracycline" only denotes the specific, preferred compound (4S,4aS,5aS,12aS)-4-(dimethylamino)-1,4,4a,5,5a,6,11,12a-octahydro-3,6,10,12,12a-pentahydroxy-6-methyl-1,11-dioxo-2-naphthacenecarboxamide:

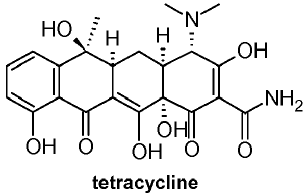

Preferably, the tetracycline compound (TC) is selected from minocycline, doxycycline, tetracycline, chlortetracycline, oxytetracycline, rolitetracycline, tigecycline, demeclocycline, lymecycline, meclocycline, methacycline, omadacycline, sarecycline, eravacycline, clomocycline, 9-(N,N-dimethylglycylamido)-6-demethyl-6-deoxytetracycline and 9-(N,N-dimethylglycylamido)-minocycline. These antibiotics have the advantage of being safe, clinically established and thus reliable and likely to be suitable for various therapeutic applications as described herein.

Most preferably, the tetracycline compound is minocycline (7-dimethylamino-6-demethyl-6-deoxytetracycline or (4S,4aS,5aR,12aR)-4,7-bis(dimethylamino)-1,10,11,12a-tetrahydroxy-3,12-dioxo-4a,5,5a,6-tetrahydro-4H-tetracene-2-carboxamide) or doxycycline (6-deoxy-5-hydroxytetracycline or (4S,4aR,5S,5aR,6R,12aR)-4-(dimethylamino)-1,5,10,11,12a-pentahydroxy-6-methyl-3,12-dioxo-4a,5,5a,6-tetrahydro-4H-tetracene-2-carboxamide), and yet further preferably minocycline.

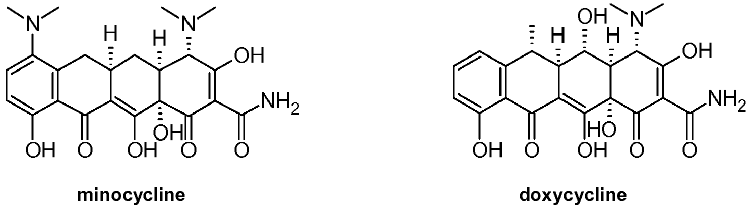

These antibiotics have the additional advantage that their applicability in the treatment of periodontal diseases is clinically established.

### Molar Ratio

The molar ratio TC : MA₂ is preferably in the range of 1 : 0.8-3.0. The present inventors found that the predominate species obtained when preparing the complexes according to the present invention are those wherein TC : MA₂ is 1:1 or 1:2. Thus, it is intended that also mixtures wherein the molar ratio TC : MA₂ is between 1:1 and 1:2 are also within the scope of the present invention. A minor excess of TC, e.g., wherein TC : MA₂ = 1 : 0.8, is also within the scope of the present invention. Similarly, an excess of MA₂, e.g., beyond a ratio of TC : MA₂ of 1:2 or even 1:3, is also considered to be within the scope of the present invention, regardless of whether the excess metal cations are coordinated to the TC. Thus, also such mixtures containing complexes with a defined ratio of TC : MA₂ together with a certain excess of one of the components are also intended to be covered by the definition of the complex according to the present invention. Preferably, TC: MA₂ is 1:1 or 1:2; more preferably TC: MA₂ is 1:2.

### Carboxylate Anion

A is a carboxylate anion derived from a C₈-C₂₄ carboxylic acid. The lipophilic nature of the carboxylate anion contributes to improving the sustained release of the tetracycline antibiotic from the complexes and preparations according to the present invention.

The carboxylic acid can be saturated or unsaturated. Preferably, A is a carboxylate anion derived from a carboxylic acid selected from arachidic acid (C₂₀), stearic acid (C₁₈), palmitic acid (C₁₆), myristic acid (C₁₄), lauric acid (C₁₂), capric acid (C₁₀), caprylic acid (C₈), hydroxystearic acid, oleic acid, linoleic acid, linolenic acid and ricinoleic acid. The hydroxystearic acid can be preferably 10-hydroxystearic acid or 12- hydroxystearic acid. Most preferably, the carboxylate anion is stearate (C₁₈).

### Pharmaceutically Acceptable Salts, Hydrates and Solvates

As used herein, the term "pharmaceutically acceptable" embraces both human and veterinary use. For example, the term "pharmaceutically acceptable" embraces a veterinarily acceptable compound or a compound acceptable in human medicine and health care. Salts, hydrates and solvates of the tetracycline compounds are those wherein the counter-ion or associated solvent is pharmaceutically acceptable. However, salts, hydrates and solvates having non-pharmaceutically acceptable counter-ions or associated solvents are within the scope of the present invention, for example, for use as intermediates in the preparation of other compounds and their pharmaceutically acceptable salts, hydrates and solvates. Suitable salts include those formed with either organic and inorganic acids or bases. Pharmaceutically acceptable acid addition salts include those formed from hydrochloric, hydrobromic, sulfuric, nitric, citric, tartaric, phosphoric, lactic, pyruvic, acetic, trifluoroacetic, triphenylacetic, sulfamic, sulfanilic, succinic, oxalic, fumaric, maleic, malic, mandelic, glutamic, aspartic, oxaloacetic, methanesulfonic, ethanesulfonic, arylsulfonic (for example p-toluenesulfonic, benzenesulfonic, naphthalenesulfonic or naphthalenedisulfonic), salicylic, glutaric, gluconic, tricarballylic, cinnamic, substituted cinnamic (for example, phenyl, methyl, methoxy or halo substituted cinnamic, including 4-methyl and 4-methoxycinnamic acid), ascorbic, oleic, naphthoic, hydroxynaphthoic (for example 1- or 3-hydroxy-2-naphthoic), naphthaleneacrylic, benzoic, 4-methoxybenzoic, 2- or 4-hydroxybenzoic, 4-chlorobenzoic, 4-phenylbenzoic, benzeneacrylic (for example 1,4-benzenediacrylic), isethionic acids, perchloric, propionic, glycolic, hydroxyethanesulfonic, pamoic, cyclohexanesulfamic, salicylic, saccharinic and trifluoroacetic acid. Particularly preferred acid addition salts are hydrochloride, hyclate (hydrochloride hemiethanolate hemihydrate, ·HCl·½EtOH·½H₂O) and acid addition salts formed from maleic or oxalic acid. Pharmaceutically acceptable base salts include ammonium salts, alkali metal salts such as those of sodium and potassium, alkaline earth metal salts such as those of calcium and magnesium and salts with organic bases such as dicyclohexylamine and *N*-methyl-*D*-glucamine. All pharmaceutically acceptable acid addition salt forms of the compounds of the present invention are intended to be embraced by the scope of the present invention.

In a preferred embodiment, the ratio TC: MA₂ is 1:2; and/or M is selected from Mg²⁺ and Ca²⁺; and/or A is a carboxylate anion derived from a carboxylic acid selected from arachidic acid (C₂₀), stearic acid (C₁₈), palmitic acid (C₁₆), myristic acid (C₁₄), lauric acid (C₁₂), capric acid (C₁₀), caprylic acid (C₈), hydroxystearic acid, oleic acid, linoleic acid, linolenic acid and ricinoleic acid.

In a further preferred embodiment, the complex has the formula [(TC)·2(MgA₂)], wherein the tetracycline compound (TC) is minocycline or a pharmaceutically acceptable salt thereof. Preferably, TC is minocycline free base or minocycline hydrochloride, and A is stearate.

### Pharmaceutical Preparations

The present invention provides a pharmaceutical preparation comprising the complex as described above and optionally one or more pharmaceutically acceptable excipient(s).

As used herein, the term "pharmaceutical preparation" is intended to encompass a product comprising the claimed compounds in the therapeutically effective amounts, as well as any product that results, directly or indirectly, from combinations of the claimed compounds.

In the pharmaceutical preparation according to the present invention, the total content of the tetracycline compound (TC) is preferably in the range of 5-30 wt.%, more preferably 8-28 wt.%, further preferably 10-20 wt.%, yet further preferably 11-15 wt.%, and most preferably 11.5 wt.% or 27.9 wt.%

### Excipients

As used herein, the term "excipient" refers to a carrier, a binder, a disintegrator and/or a further suitable additive for a galenic formulation, such as extrudate, cream, gel, emulsion, suspension, liniment, ointment, powder, paste, balm, lotion, eye drops, spray, topical aerosol, topical solution, topical suspension, skin patch and nonwoven. Carriers, which can be added to the mixture, include necessary and inert pharmaceutical excipients, including, but not limited to, suitable release retardants, suspending agents, lubricants, flavorants, sweeteners, preservatives, coatings, granulating agents, dyes, and coloring agents.

A particularly preferable class of excipients are biodegradable polymers, which will be discussed in greater detail below.

Further preferable types of excipients are emulsifiers, such as glycerol monostearate, which may be present preferably in an amount of 5 to 15 wt.%, more preferably 8 to 12 wt.%, most preferably 10 wt.%. By adding such excipients, the release behavior of the preparations, in particular extrudates, can be modulated (e.g., accelerated).

Further preferable types of excipients are plasticizers, such as polyethylene glycol (PEG), which may be present preferably in an amount of 5 to 15 wt.%, more preferably 8 to 12 wt.%, most preferably 10 wt.%. A preferable type of plasticizer is PEG 1500. By adding such excipients, the mechanical behavior of the preparations, in particular of extrudates, can be modulated (e.g., brittleness and/or a tendency towards hardening over time can be reduced).

### Routes of Administration and Types of Preparations

Preferably, the pharmaceutical preparation according to the present invention is arranged for topical administration. Topical administration means application to body surfaces such as the skin or mucous membranes to treat ailments via a large range of classes including creams, foams, gels, lotions, and ointments. Many topical medications are epicutaneous, meaning that they are applied directly to the skin. Topical medications may also be inhalational, such as asthma medications, or applied to the surface of tissues other than the skin, such as eye drops applied to the conjunctiva, or ear drops placed in the ear, or medications applied to the surface of a tooth, gum or periodontal pocket.

Further preferably, the pharmaceutical preparation is selected from the group consisting of extrudate, particle, granule, powder, film, strip, compact, chip, paste, cream, gel, emulsion, suspension, liniment, ointment, balm, lotion, eye drops, spray, topical aerosol, topical solution, topical suspension, skin patch and nonwoven. Yet further preferably, the pharmaceutical preparation is arranged for application into a periodontal pocket.

### Extrudates

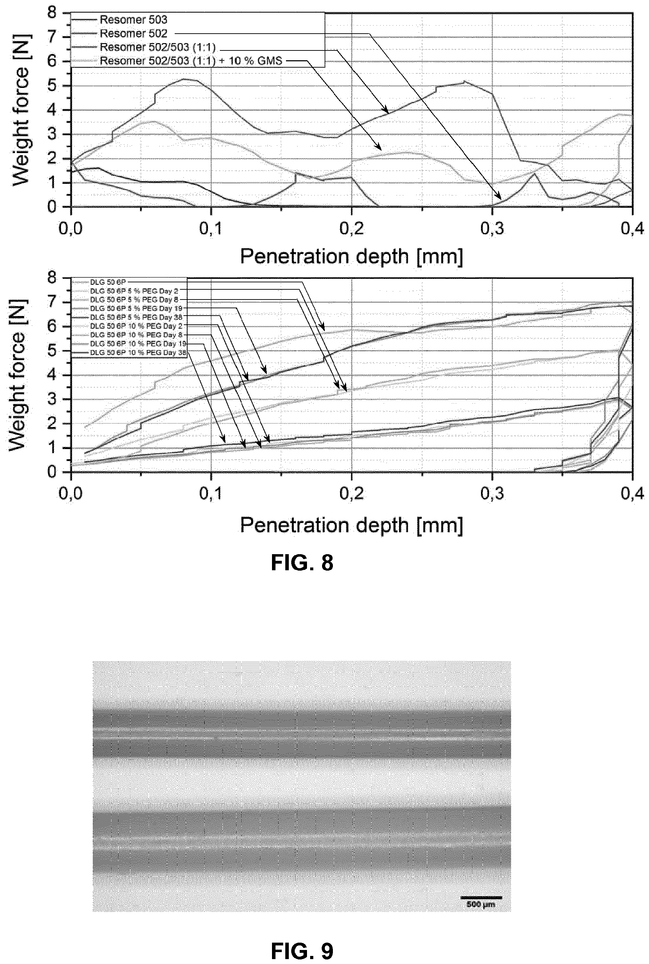

As used herein, the term "extrudate" refers to any material that has been extruded through a die. Specifically, extrudates refer to dosage forms obtained by shaping a precursor by extrusion into rods which can be either cut into pieces or used as such, e.g. as a sustained release dosage form for topical or a different route of administration. Preferably, the pharmaceutical preparation is in the form of a strand-shaped extrudate, e.g., as shown in Fig. 9. The strand-shaped extrudate preferably has an essentially circular or an essentially elliptical cross section. and/or a maximum cross-sectional diameter in the range of 0.1-10 mm, more preferably 0.2-5 mm, further preferably 0.3-1 mm, yet further preferably 0.4-0.8 mm, most preferably 0.6 mm. A suitable diameter allows for easy, pain-free and reliable placement into a periodontal pocket of a given size.

### Biodegradable Polymers

The pharmaceutical preparation according to the present invention preferably comprises, as a possible excipient, one or more biodegradable polymer(s). These may be selected from biodegradable polyesters, such as poly(glycolic acid) (PGA), poly(lactic acid) (PLA), poly(lactic-co-glycolic acid) (PLGA), polyhydroxybutyric acid (PHB) or polycaprolactone (PCL); mixed biodegradable polyesters, such as PLA-PCL and PLGA-PCL; biodegradable PEGylated diblock (AB) or triblock (ABA or BAB) copolymers, such as PEG-PLA, PEG-PLGA, PEG-PCL and PEG-PCL-PLGA; and pectins,

Poly(glycolic acid) (PGA), Poly(lactic acid) (PLA) and their copolymers are biodegradable polyesters suitable for use the present invention. They degrade in the body by simple hydrolysis of the ester backbone to non-harmful and non-toxic compounds. The degradation products are either excreted by the kidneys or eliminated as carbon dioxide and water through well-known biochemical pathways. Since PLA/PGA polymers are considered safe, non-toxic and biocompatible by regulatory agencies in virtually all developed countries, additional applications of these materials can be brought to market sooner and are more cost effective than those utilizing novel polymers with unproven biocompatibility. Examples of PLA/PGA polymers suitable for use in the present invention are for instance the RESOMER polymers manufactured by Evonik Röhm Pharma GmbH shown in Table 1 below (*RESOMER*® *Biodegradable Polymers for Medical Device Applications Research. RESOMER*® *Materials by Evonik Röhm GmbH,* https://www.sigmaaldrich.com/technical-documents/articles/materials-science/polymer-science/resomer.html, retrieved 25.10.2018).

### Poly(glycolic acid) (PGA),

PGA is a highly crystalline material with a high melting point (225-230 °C) and variable solubility in organic solvents that is generally low and depends on polymer molecular weight. It is still susceptible to hydrolysis due to the ester bond in the polymer backbone. In spite of its low solubility, this polymer has been fabricated into a variety of forms and structures. Techniques used to develop PGA-based structures include extrusion, injection and compression molding as well as particulate leaching and solvent casting. Fibers of PGA have a high strength and modulus and are particularly rigid.

### Poly(lactic acid) (PLA)

Unlike glycolide, lactide is a chiral molecule and exists in two distinct optically active forms - L-lactide and D-lactide. When each of these monomers is polymerized, the resulting polymer is semi-crystalline. Polymerization of a racemic mixture of L- and D-lactides forms poly-D,L-lactide (PDLLA), which is amorphous and has a glass transition temperature of 55-60°C. The degree of crystallinity can be tuned by altering the ratio of D to L enantiomers within the polymer. Selection of the PLA stereochemistry can have a major effect on the polymer properties, processability and biodegradability. Poly (L-lactide) or PLLA is often the polymer of choice for cast/extruded biomedical devices because it breaks down into L(+)-lactic acid units, which is the naturally occurring stereoisomer and is therefore excreted with minimal toxicity.

### Poly (Lactide-co-Glycolide) Copolymers (PLGA)

Both L- and DL-lactides have been used for co-polymerization. The ratio of glycolide to lactide at different compositions allows control of the degree of crystallinity of the polymers. When the crystalline PGA is co-polymerized with PLA, the degree of crystallinity is reduced and as a result this leads to increases in rates of hydration and hydrolysis. It can therefore be concluded that the degradation time of the copolymer is related to the ratio of monomers used in synthesis. In general, the higher the content of glycolide, the quicker the rate of degradation. However, an exception to this rule is the 50:50 ratio of PGA: PLA, which exhibits the fastest degradation.

As end groups, three types of end-groups functional groups are preferred: (i) free carboxylic acid group, (ii) ester terminated group, (iii) alkyl ester group. Polymers capped with ester terminated and alkyl ester groups typically show longer degradation lifetimes than the free carboxylic analogs.

**Table 1: RESOMER® Biodegradable Polymers**

| **Product No.** | **RESOMER Type** | **Product Name** | **Molecular Weight Range** | **Viscosity [dl/g]** | **Application** | **Tg [°C]** | **Tm [°C]** | **End Group** |
|---|---|---|---|---|---|---|---|---|
| 719854 | RESOMER L 206 S | Poly(L-lactide) | - | 0.8-1.2 | Medical Device | 60-65 | 180-185 | ester terminated |
| 719951 | RESOMER R 202 S | Poly(D,L-lactide) | 10,000- 18.000 | 0.16-0.24 | Controlled Release | 38-42 | amorphous | ester terminated |
| 719978 | RESOMER R 202 H | Poly(D,L-lactide) | 10,000- 18.000 | 0.16-0.24 | Controlled Release | 44-48 | amorphous | free carboxylic |
| 719935 | RESOMER R 203 S | Poly(D,L-lactide) | 18,000- 28,000 | 0.25-0.35 | Controlled Release | 46-50 | amorphous | ester terminated |
| 719943 | RESOMER R 203 H | Poly(D,L-lactide) | 18,000- 28,000 | 0.25-0.35 | Controlled Release | 48-52 | amorphous | free carboxylic |
| 719889 | RESOMER RG 502 | Poly(D,L-lactide-co- | 7,000- 17.000 | 0.16-0.24 | Controlled Release | 42-46 | amorphous | alkyl ester |
| 719897 | RESOMER RG 502 H | Poly(D,L-lactide-co- | 7,000- 17.000 | 0.16-0.24 | Controlled Release | 42-46 | amorphous | free carboxylic |
| 739952 | RESOMER RG 503 | Poly(D,L-lactide-co- | 24,000 - 38.000 | 0.32-0.44 | Controlled Release | 44-48 | amorphous | ester |
| 719870 | RESOMER RG 503 H | Poly(D,L-lactide-co- | 24,000- 38.000 | 0.32-0.44 | Controlled Release | 44-48 | amorphous | free carboxylic |
| 739944 | RESOMER RG 504 | Poly(D,L-lactide-co- | 38,000 - 54,000 | 0.45-0.60 | Controlled Release | 46-50 | amorphous | ester |
| 719900 | RESOMER RG 504 H | Poly(D,L-lactide-co- | 38,000- 54,000 | 0.45-0.60 | Controlled Release | 46-50 | amorphous | free carboxylic |
| 739960 | RESOMER RG 505 | Poly(D,L-lactide-co- | 54,000 - 69.000 | 0.61-0.74 | Controlled Release | 48-52 | amorphous | ester |
| 719862 | RESOMER RG 653 H | Poly(D,L-lactide-co- | 24,000- 38,000 | 0.32-0.44 | Controlled Release | 46-50 | amorphous | free carboxylic |
| 719919 | RESOMER RG 752 H | Poly(D,L-lactide-co- | 4,000- 15.000 | 0.14-0.22 | Controlled Release | 42-46 | amorphous | free carboxylic |
| 719927 | RESOMER RG 756 S | Poly(D,L-lactide-co- | 76,000- 116,000 | 0.71-1.0 | Controlled Release | 49-55 | amorphous | ester terminated |
| 739979 | RESOMER RG 858 S | Poly(D,L- lactide-co- | 190,000 - 240,000 | 1.3-1.7 | Controlled Release | - | amorphous | alkyl ether |
| 719846 | RESOMER X | Polydioxanone | -- | 1.5-2.2 (0.1% in HFIP, 30°C) | Medical Device | (-10)-(-5) | 110-115 | -- |
| | *Inherent Viscosity [dl/g], (0.1% in CHCl₃, 25°C, except where noted) | | | | | | | |

Further suitable biodegradable polymers for use in the present invention are available under the name EXPANSORB® from Merck KGaA *(PLA & PLGA Polymer Portfolio for Peptide & Small Molecule Formulation* http://www.merckmillipore.com/INTERSHOP/web/WFS/Merck-DE-Site/de_DE/-/EUR/ShowDocument-Pronet?id=201604.101, retrieved 25.10.2018):

**Table 2: EXPANSORB® Biodegradable Polymers**

| **Product** | **Order No.** | **Poly (D,L-lactic acid) (PLA)** | **Ratio L/DL** | **End group** | **I.V. range (dL/g, CHCl₃,25°C)** | **MW (indicative range, kDa)** |
|---|---|---|---|---|---|---|
| EXPANSORB® DL 100-1A | 5.43108 | PDLLA | 0/100 | COOH | 0.05-0.20 | 6-20 |
| EXPANSORB® DL 100-2A | 5.43109 | PDLLA | 0/100 | COOH | 0.15-0.30 | 10-25 |
| EXPANSORB® DL 100-5A | 5.43110 | PDLLA | 0/100 | COOH | 0.40-0.60 | 45-80 |
| EXPANSORB® DL 100-7A | 5.43111 | PDLLA | 0/100 | COOH | 0.60-0.80 | 70-120 |
| EXPANSORB® DLL 10-15A | 5.43112 | P(L/DL)LA | 90/10 | COOH | 1.30-1.70 | 200-250 |
| EXPANSORB® DL 100-2E | 5.43113 | PDLLA | 0/100 | ESTER | 0.15-0.30 | 10-25 |

| **Product** | **Order No.** | **Poly(D,L-lactic-co- glycolic acid) (PLGA)** | **Ratio L/DL** | **End group** | **I.V. range (dL/g, CHCl₃, 25°C)** | **MW (indicative range, kDa)** |
|---|---|---|---|---|---|---|
| EXPANSORB® DLG 45-2A | 5.43114 | PLGA | 45/55 | COOH | 0.15-0.30 | 15-30 |
| EXPANSORB® DLG 50-2A | 5.43115 | PLGA | 50/50 | COOH | 0.15-0.25 | 5-20 |
| EXPANSORB® DLG 50-3A | 5.43116 | PLGA | 50/50 | COOH | 0.25-0.40 | 15-40 |
| EXPANSORB® DLG 50-5A | 5.43117 | PLGA | 50/50 | COOH | 0.40-0.55 | 42-65 |
| EXPANSORB® DLG 50-6A | 5.43118 | PLGA | 50/50 | COOH | 0.55-0.65 | 60-85 |
| EXPANSORB® DLG 50-8A | 5.43119 | PLGA | 50/50 | COOH | 0.65-0.90 | 80-130 |
| EXPANSORB® DLG 55-5A | 5.43120 | PLGA | 55/45 | COOH | 0.40-0.50 | 42-65 |
| EXPANSORB® DLG 65-3A | 5.43121 | PLGA | 65/35 | COOH | 0.25-0.35 | 15-40 |
| EXPANSORB® DLG 65-6A | 5.43122 | PLGA | 65/35 | COOH | 0.50-0.65 | 45-85 |
| EXPANSORB® DLG 75-2A | 5.43124 | PLGA | 75/25 | COOH | 0.08-0.21 | 5-20 |
| EXPANSORB® DLG 75-5A | 5.43125 | PLGA | 75/25 | COOH | 0.38-0.64 | 37-84 |
| EXPANSORB® DLG 75-7A | 5.43126 | PLGA | 75/25 | COOH | 0.60-0.70 | 65-85 |
| EXPANSORB® DLG 75-8A | 5.43127 | PLGA | 75/25 | COOH | 0.70-0.90 | 76-130 |
| EXPANSORB® DLG 75-10A | 5.43123 | PLGA | 75/25 | COOH | 0.80-1.10 | 110-166 |
| EXPANSORB® DLG 85-2A | 5.43129 | PLGA | 85/15 | COOH | 0.15-0.25 | 10-15 |
| EXPANSORB® DLG 85-7A | 5.43128 | PLGA | 85/15 | COOH | 0.55-0.75 | 65-95 |
| EXPANSORB® DLG 90-5A | 5.43130 | PLGA | 90/10 | COOH | 0.35-0.55 | 30-70 |
| EXPANSORB® DLG 90-7A | 5.43131 | PLGA | 90/10 | COOH | 0.60-0.75 | 50-95 |
| EXPANSORB® DLG 95-2A | 5.43132 | PLGA | 95/5 | COOH | 0.15-0.25 | 5-20 |
| EXPANSORB® DLG 95-4A | 5.43133 | PLGA | 95/5 | COOH | 0.25-0.50 | 35-60 |
| EXPANSORB® DLG 50-2E | 5.43134 | PLGA | 50/50 | ESTER | 0.15-0.25 | 5-20 |
| EXPANSORB® DLG 50-6E | 5.43135 | PLGA | 50/50 | ESTER | 0.55-0.65 | 60-85 |
| EXPANSORB® DLG 50-7E | 5.43136 | PLGA | 50/50 | ESTER | 0.60-0.70 | 70-100 |
| EXPANSORB® DLG 65-6E | 5.43137 | PLGA | 65/35 | ESTER | 0.50-0.65 | 45-85 |
| EXPANSORB® DLG 75-2E | 5.43138 | PLGA | 75/25 | ESTER | 0.15-0.30 | 10-25 |
| EXPANSORB® DLG 75-4E | 5.43139 | PLGA | 75/25 | ESTER | 0.30-0.50 | 25-60 |
| EXPANSORB® DLG 75-7E | 5.43140 | PLGA | 75/25 | ESTER | 0.66-0.80 | 80-115 |
| EXPANSORB® DLG 75-9E | 5.43141 | PLGA | 75/25 | ESTER | 0.75-1.00 | 100-160 |
| EXPANSORB® DLG 85-7E | 5.43142 | PLGA | 85/15 | ESTER | 0.55-0.75 | 65-95 |
| EXPANSORB® DLG 85-12E | 5.45583 | PLGA | 85/15 | ESTER | 1.00-1.30 | 185-210 |
| EXPANSORB® DLG 50-6P | 5.43143 | PLGA-PEG | 50/50 | PEG | 0.45-0.65 | 30-60 |
| EXPANSORB® DLG 50-7P | 5.43144 | PLGA-PEG | 50/50 | PEG | 0.65-0.80 | 60-85 |
| EXPANSORB® DLG 75-4A | 5.45223 | PLGA | 75/25 | COOH | 0.30-0.50 | 25-60 |

Preferred biodegradable polymers are poly(D,L-lactide-co-glycolides) with a PLA/PGA ratio of 50:50; preferably having a weight average molecular weight range of 7,000-38,000, more preferably 7,000-17,000 or 24,000-38,000; and/or an intrinsic viscosity of 0.16-0.44, more preferably 0.16-0.24 or 0.32-0.44; and/or a glass transition temperature of 42-48 °C, more preferable 42-46 or 44-48 °C. Further preferably, the biodegradable polymer is selected from Resomer® RG 502 ("Resomer 502"), Resomer® RG 503 ("Resomer 503") or a mixture thereof.

Preferred biodegradable polymers are also poly(D,L-lactide-co-glycolides) with a PLA/PGA ratio of 50:50 and a PEG end group (PLGA-PEG); preferably having a weight average molecular weight range of 30-60 kDa; and/or an intrinsic viscosity of 0.45-0.65. Further preferably, the biodegradable polymer is EXPANSORB® DLG 50-6P.

### Methods for Manufacturing

The present invention further provides a method for manufacturing the complex as described above, the method comprising the following steps: (a) mixing a tetracycline compound (TC) or a pharmaceutically acceptable salt, hydrate or solvate thereof and a divalent metal carboxylate (MA₂) in a molar ratio of 1 : 0.8-3.0 with an organic solvent to obtain a dispersion, wherein the divalent metal cation M is an earth alkaline metal cation and A is a carboxylate anion derived from a C₈-C₂₄ carboxylic acid; (b) heating said dispersion to form the complex; (c) removing the solvent to obtain the complex. Preferable tetracycline compounds, molar ratios, carboxylate anions, pharmaceutically acceptable salts, hydrates and solvates are as described in the section "Complexes" above. In a preferred embodiment, the complex according to the present invention is obtained/obtainable by a method for manufacturing as described herein.

In the mixing step (a), a preferable solvent is, e.g., an alcohol, such as methanol, ethanol or mixtures thereof in the presence or in the absence of water. Preferably, a mass/volume ratio of TC to solvent of 1:100 to 1:300, preferably 1:200 may be used.

Heating the dispersion (b) accelerates the formation of the complex, preferably at a temperature of 50 °C or more, more preferably 60 °C or most preferably 70 °C or more and up to the boiling point of the solvent. Depending on the solvent chosen, the temperature and the mass/volume ratio of TC to solvent, complete dissolution may occur during step (b), such that a solution of the complex is obtained.

In step (c), the solvent can be removed by a suitable method known in the art. Preferably, the solvent may be evaporated at room temperature or elevated temperature; and/or in a shaker, an oven or on/in a film ; and/or under atmospheric or preferably reduced pressure.

The pharmaceutical preparations according to the present invention, unless otherwise specified, may be prepared according to a suitable method known in the art. However, when the pharmaceutical preparations are in the form of strand-shaped extrudates, the latter are preferably prepared according to a method for comprising the following steps: (d) comminuting said complex and, if present, one or more pharmaceutically acceptable excipients to obtain an extrusion precursor; (e) extruding said extrusion precursor at a temperature above room temperature; (f) cooling the product of step (e) to obtain the pharmaceutical preparation in the form of a strand-shaped extrudate. preferred excipients, routes of administration and types of preparations, extrudates, and biodegradable polymers are as described in the section "Pharmaceutical Preparations" above. In a preferred embodiment, the pharmaceutical preparation according to the present invention is obtained/obtainable by a method for manufacturing as described herein.

In the comminution step (d), the complex is mixed, comminuted and homogenized together with one or more pharmaceutically acceptable excipient(s) (as described above, and if present) to obtain an extrusion precursor. Preferably, one or more biodegradable polymer(s), emulsifier(s) and/or plasticizer(s) as described above may be added as excipients. Further preferably, the content of the tetracycline compound (TC) added is in the range of 5-30 wt.%, more preferably 8-28 wt.%, further preferably 10-20 wt.%, yet further preferably 11-15 wt.%, and most preferably 11.5 wt.% or 27.9 wt.% relative to the total amount of components mixed. Comminution is preferably performed by milling or grinding, preferably in a mill or a grinder, more preferably at a low temperature, e.g., in a cryomill at -196 °C. When a cryomill is used, performing 6, preferably 5 milling cycles at a frequency of 30 Hz for 150 s with intermediate cooling (30 s) at a frequency of 5 Hz between the cycles is particularly preferred to obtain an extrusion precursor.

The extrusion step (e) is performed by extruding the extrusion precursor through a die. Preferably, a twin-scree extruder may be used. The extrusion is performed at a suitable temperature above room temperature which is sufficient to ensure plastic deformability of the extrusion precursor material. Depending on the composition of the precursor, this is preferably a temperature about equal to or above the softening point (in the case of a pure complex without polymer ingredients); or to or above the glass transition temperature ((in the case where polymer ingredient, such as biodegradable polymers are present). Typically, the extrusion temperature may be preferably in the range of 48 to 56 °C, more preferably from 49 to 55 °C, even more preferably selected from 49, 52, 53 and 55 °C. The cross-sectional shape of the extrudate is mainly determined by the die at the exit of the extruder, which may be preferably essentially circular or an essentially elliptical cross section and/or a maximum cross-sectional diameter in the range of 0.1-10 mm, more preferably 0.2-5 mm, further preferably 0.3-1 mm, yet further preferably 0.4-0.8 mm, most preferably 0.6 mm. The die of the extruder can be exchanged as desired by a plate with different diameters or geometric forms.

Upon cooling (f) of the extruded product of step (e), a pharmaceutical preparation in the form of a strand-shaped extrudate is obtained, as shown, e.g., in Fig. 9. The thus obtained strand-shaped may be further cut into smaller pieces or deformed as desired, e.g. in order to be adopted for placement into a periodontal pocket of a certain size or shape.

### Therapeutic Applications

The present invention further provides a complex and/or a pharmaceutical preparation as defined above for use in a method for treatment of the human or animal body. The present disclosure also provides a method for treatment of the human or animal body wherein the method comprises administration of a therapeutically effective amount of a complex and/or a pharmaceutical preparation as defined above to a subject in need thereof.

The present invention further provides a complex and/or a pharmaceutical preparation as defined above for use in a method for therapy and/or prophylaxis of a bacterial infection, preferably caused by one or more bacteria selected from the group consisting of *Porphyromonas gingivalis, Prevotella intermedia, Tannerella forsythia, Streptococcus gordonii, Fusobacterium nucleatum, Actinomyces naeslundii* and *Parvimonas micra;* and/or wherein antibiotic activity is maintained over a period of at least 21 days, preferably at least 25, more preferably at least 28, further more preferably at least 35 days, most preferably 42 days; and/or for use in a method for therapy and/or prophylaxis of an acute, chronic or recurrent periodontal disease. The disclosure further provides a method for therapy and/or prophylaxis of a bacterial infection, preferably caused by one or more bacteria selected from the group consisting of *Porphyromonas gingivalis, Prevotella intermedia, Tannerella forsythia, Streptococcus gordonii, Fusobacterium nucleatum, Actinomyces naeslundii* and *Parvimonas micra;* and/or wherein antibiotic activity is maintained over a period of at least 21 days preferably at least 25, more preferably at least 28, further more preferably at least 35 days, most preferably 42 days; and/or a method for therapy and/or prophylaxis of an acute, chronic or recurrent periodontal disease, each comprising administration of a therapeutically effective amount of a complex and/or a pharmaceutical preparation as described above to a subject in need thereof, respectively.

It is currently considered that *P. gingivalis, P. intermedia, T. forsythia, F. nucleatum and P. micros* are pathogens particularly associated with periodontitis. Additionally, *A. naeslundii* and S. *gordonii,* belonging to the so-called "early colonizers", are considered to be essential for the formation of a biofilm (e.g., an oral periopathogenic biofilm) and are also present in the cases of periodontal diseases.

The major categories of periodontal diseases and conditions are classified in the groups of dental plaque-induced gingival diseases, chronic periodontitis, aggressive periodontitis, periodontitis as a manifestation of systemic diseases, necrotizing periodontal diseases, abscesses of the periodontium, periodontitis associated with endodontic lesions, peri-implant mucositis, peri-implantitis, and endodontic infections. In the present invention, the acute, chronic or recurrent periodontal disease is preferably selected from the group consisting of dental plaque-induced gingival diseases, periodontitis, chronic periodontitis, aggressive periodontitis, periodontitis as a manifestation of systemic diseases, necrotizing periodontal diseases, abscesses of the periodontium, periodontitis associated with endodontic lesions, peri-implant mucositis, peri-implantitis and endodontic infections. The present disclosure also provides a method for therapy or prophylaxis of an acute, chronic or recurrent periodontal disease which is preferably selected from the group consisting of dental plaque-induced gingival diseases, periodontitis, chronic periodontitis, aggressive periodontitis, periodontitis as a manifestation of systemic diseases, necrotizing periodontal diseases, abscesses of the periodontium, periodontitis associated with endodontic lesions, peri-implant mucositis, peri-implantitis and endodontic infections, wherein the method comprises administration of a therapeutically effective amount of a complex and/or a pharmaceutical preparation as described above to a subject in need thereof. Further acute, chronic or recurrent periodontal diseases are described, e.g., in Armitage, A. Ann Periodontol 1999, 4, 1-6.

The term "subject" as used herein refers to an animal, preferably a mammal, most preferably a human, who is or has been the object of treatment, therapy, prophylaxis, observation or experiment.

The term "therapeutically effective amount" as used herein means that amount of active compound or pharmaceutical agent that elicits the biological or medicinal response in a tissue system, animal or human being sought by a researcher, veterinarian, medical doctor or other clinician, which includes alleviation of the symptoms of the disease or disorder being treated.

### EXAMPLES

### Formation and properties of complexes

### Example 1: Formation of a 1:2 complex of minocycline (free base) and magnesium stearate

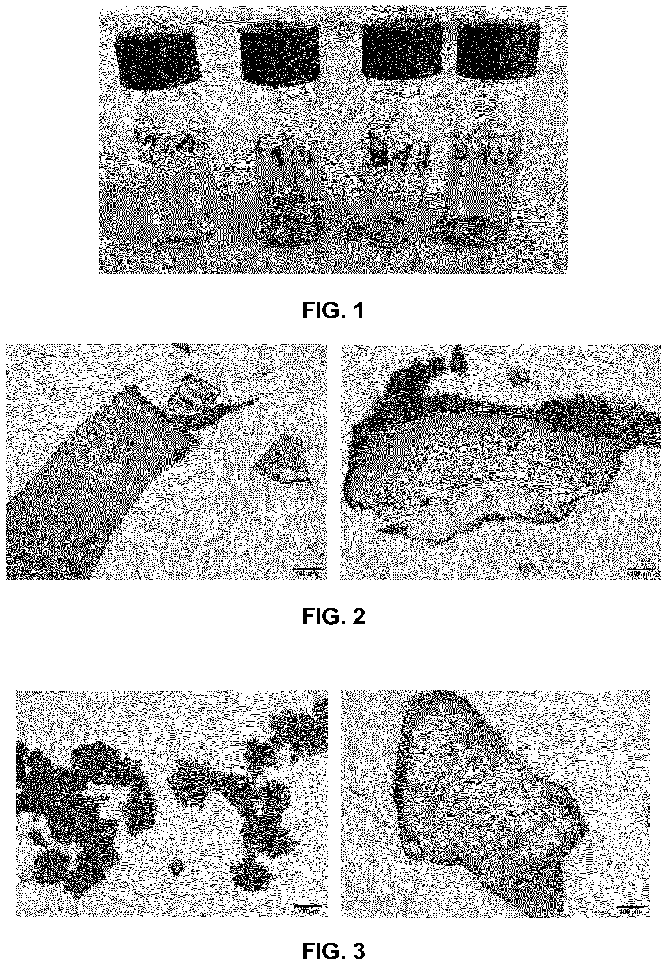

Minocycline (free base) was weight in a round-bottom flask together with magnesium stearate in a molar ratio of 1:2. Specifically, minocycline (10 mg, M = 457.48 g/mol, 218.8 µmol) was combined with magnesium stearate (25.848 mg, M = 591.24 g/mol, 43.717 µmol). Both components were briefly mixed in a hand-shaker. The mixture was suspended in undenaturated ethanol (96% v/v, 2 mL) to afford a light yellow, non-translucent suspension. The closed flask was placed in a water bath under gentle swirling. Beginning at about 50°C, formation and dissolution of the complex started. At about 60-70°C, a clear, orange solution was formed which remained clear upon cooling to room temperature. The solvent was removed in a tempered shaker. Fig. 1 shows vials with the dried products.

### Example 2: Formation of a 1:1 complex of minocycline (free base) and magnesium stearate

A complex having a molar ratio of 1:1 was prepared in an analogous way as described in Example 1 by using an appropriately adapted amount of minocycline (free base).

### Example 3: Formation of a 1:2 complex of minocycline hydrochloride and magnesium stearate

A complex of minocycline hydrochloride and magnesium stearate having a molar ratio of 1:2 was prepared in a similar manner as described in Example 1 above, wherein an appropriate amount of minocycline hydrochloride was used such that the molar ratio of minocycline and magnesium stearate was 1:2.

### Example 4: Formation of a 1:1 complex of minocycline hydrochloride and magnesium stearate

A complex of minocycline hydrochloride and magnesium stearate having a molar ratio of 1:1 was prepared in a similar manner as described in Example 1 above, wherein an appropriate amount of minocycline hydrochloride was used such that the molar ratio of minocycline and magnesium stearate was 1:1.

### Example 5: Formation of a 1:2 complex of doxycycline (free base) and magnesium stearate

A complex of doxycycline (free base) and magnesium stearate having a molar ratio of 1:2 was prepared in a similar manner as described under Example 1 above, wherein doxycycline was used instead of minocycline.

### Example 6: Formation of a 1:1 complex of doxvcvcline (free base) and magnesium stearate

A complex of doxycycline (free base) and magnesium stearate having a molar ratio of 1:1 was prepared in a similar manner as described under Example 1 above, wherein however doxycycline free base was used instead of minocycline an appropriate amount of doxycycline was such that the molar ratio was 1:1.

The products from the above examples have different properties as compared to those of the respective tetracycline compounds (minocycline, minocycline hydrochloride and doxycycline), which is indicative for the formation of distinct, new complexes.

The solubility of the complexes as compared to the respective tetracycline compound (e.g., pure minocycline) is reduced. Minocycline is soluble in most polar solvents, such as water, dimethyl sulfoxide, ethanol, methanol and *N*-methyl pyrrolidone. The complexes, in contrast, are sparingly soluble in water but are well-soluble in chloroform. The stearate counter ion is considered to contribute to the modified solubility properties. Variations of the counter ion may be considered in order to tune the solubility.

The products having a molar ratio of the tetracycline compound to magnesium stearate of 1:1 crystallize upon drying, whereas those having a molar ratio of 1:2 solidify to afford a glassy solid (Fig. 1). Samples were taken from these solids and studied under a microscope. In the solids having a molar ratio of 1:1, an inhomogeneous composition was observed (Fig. 2, left), whereas the samples having a molar ratio of 1:2 showed a uniform appearance (Fig. 2, right). Similarly, the solid obtained from doxycycline and magnesium stearate in a ratio of 1:1 afforded inhomogeneous agglomerates (Fig. 3, left), whereas a glassy structure was formed in case of a molar ratio of 1:2 (Fig. 3, right) extraction by means of a spatula.

### Example 7: Stabilization in Aqueous Environment

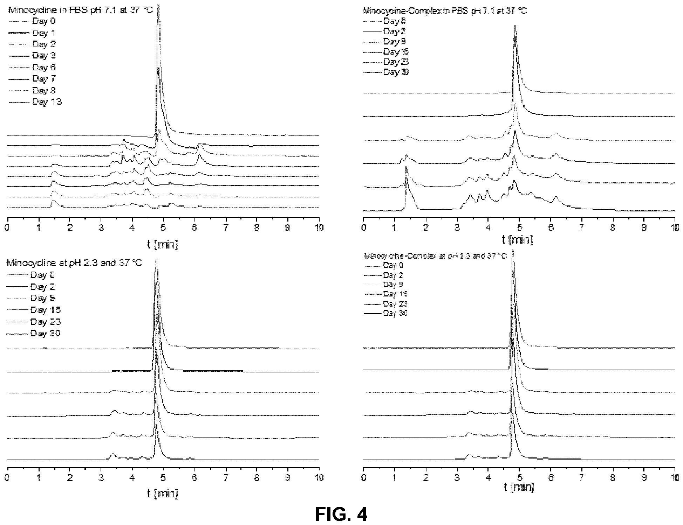

Minocycline (10 mg; Fig. 4, left hand) and the complex of Example 1 (20 mg; Fig. 4, right-hand side) were placed in separate vials to provide an approximately equal amount of minocycline. Each sample was dispersed in 4 mL of the respective buffer (PBS) at pH 7.1 (Fig. 4, top row) or pH 2.3, (Fig. 4, bottom row). The samples with the complex resulted in a suspension due to the lower solubility of the complex. The samples were incubated at 37 °C over multiple days and spectra were recorded upon separation by means of HPLC with an UV detector at a wavelength of 355 nm. The dominant peak at retention time of 4.75 min corresponds to minocycline. In the neutral pH area at 37°C, a complete decomposition is observed after 3 days in the case of pure minocycline (Fig. 4, top left). Under acidic conditions, the decomposition process is slower, although minocycline nevertheless continuously decays to some extent. The complex, in turn, is less susceptible to decomposition because it is less soluble due to its lipophilic properties and reacts less intensively with the solvent (Fig. 4, right-hand side). It is considered that during incubation, further fractions of the complex are dissolved and only then exposed to decomposition. Although decay of the active substance is then observed as well, it is nevertheless much slower in the case of the complex, in particular under neutral conditions (Fig. 4, top right). Thus, the complexation is considered to provide stabilization towards the composition in aqueous environment.

### Example 8: Spectroscopic Properties

### a) UV/Vis Absorption Spectra

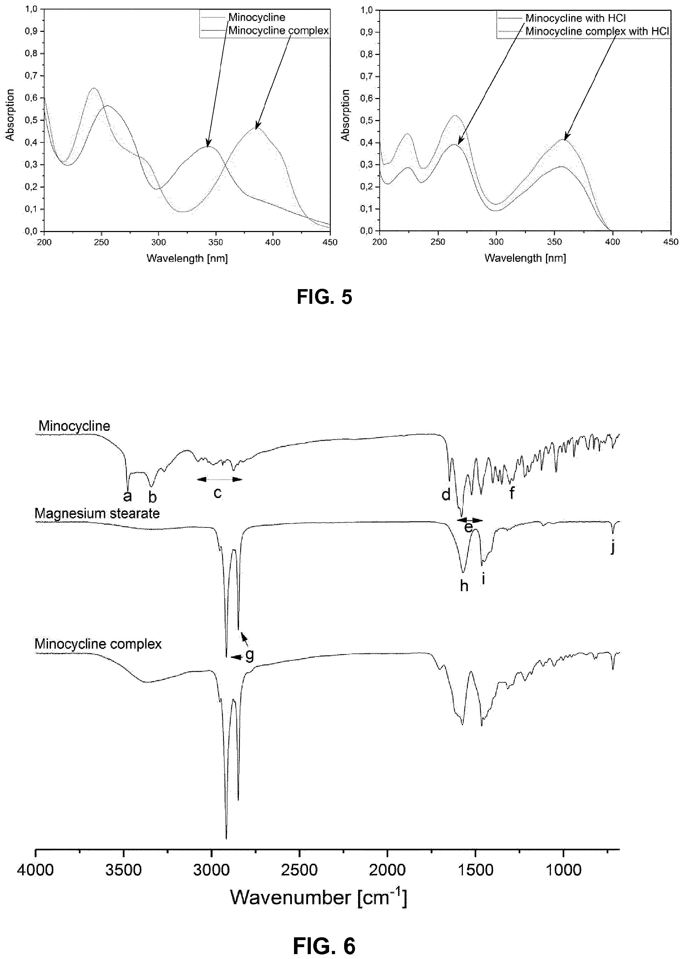

Furthermore, changes in the absorption spectra were observed (Fig. 5). Freshly prepared ethanol solution of minocycline and the complex of Example 1 were prepared at a concentration of 5 mg/mL of minocycline. From these stock solutions, 10 µL were taken and diluted with ethanol (3 mL) such that a final concentration of 16.6 µg/mL minocycline was obtained. The spectra measured by means of a Shimadzu UV-1800 spectrophotometer. Minocycline shows absorption maxima at 255 and 344 nm. Upon complexation, the first maximum is hypsochromically shifted to 244 nm, whereas the other maximum is bathochromically shifted to 385 nm. Upon addition of a small amount of hydrochloric acid, both maxima shift to 264 and 355 nm.

### b) IR Absorption Spectra

FTIR-ATR spectra were measured in potassium bromide pellets using a Bruker IFS 28 instrument equipped with a Sensir ATR unit in the area of 4000 to 400 cm⁻¹. The IR spectra as well provide indications for the complexation (Fig. 6; minocycline: a - υ O-H; b - υ N-H; c - υ C-H; d - υ C=O / CONH₂; e - υ C=C (aromatic); f - υ C-N; Magnesium stearate: g - υ C-H; h - υₐₛ COO⁻; i - δ C-H; j - CH₂ rocking-vibration). The peaks at 3478 cm⁻¹ of pure minocycline can be attributed to the vibration of the hydroxy group forming intramolecular hydrogen bonds. In contrast, the complex shows a broad peak at the same position. Due to the fact that this group forms a part of the coordination site for chelate formation, hindrance of the free vibration of the hydroxy groups and thus smoothening of the peak is observed upon complexation. Furthermore, the C-H-vibrations of minocycline are overshadowed by the intense C-H-vibrations of magnesium stearate. The carboxylate peak at 1570 cm⁻¹ remains recognizable. The newly arising peak at 1700 cm⁻¹ cannot be directly attributed to a functional group. The fingerprint area is also modified due to the overlapping vibrations.

### Formation and properties of pharmaceutical preparations (extrudates)

### Example 9: Extrusion of a 1:2 complex of minocycline (free base) and magnesium stearate

### a) Complex Preparation

For extrusion, typically a larger amount of the complexes is required (e.g., an amount corresponding to 100 mg minocycline or more). The components were weighed and treated as described above under Example 1. Subsequently, the complex solution was spread onto multiple Petri dishes coated with Teflon film, and was covered with perforated aluminum foil. The Petri dishes were dried in a vacuum dying oven at room temperature over at least 8 hours (or overnight). A yellow powder was obtained, which was then removed from the Teflon film by means of a card sheet.

### b) Extrusion Precursor Preparation

Then, comminution and homogenization was performed by means of a Retsch CryoMill. The complex powder (1.00 g, corresponding to 289 mg of pure minocycline) was weighed in the cryomill jar together with two milling balls of 1 cm size in the cryomill jar. The jar was fitted into the cryomill and the following parameters were adjusted: upon automatic precooling phase, 6 milling cycles at a frequency of 30 Hz for 150 s each were performed; between the individual milling cycles, intermediate cooling (30 s) was performed at a frequency of 5 Hz. Upon thawing up to room temperature, the extrusion precursor was obtained.

### c) Extrusion

For extrusion, the three heating zones of a twin-screw extruder were pre-heated at H1 = 49 °C, H2 = 52 °C and H3 = 55 °C. The control unit of the twin-screw extruder was set to 800 rpm, which corresponds to an actual rotation frequency of estimated 150 turns of the screws per minute. The extrusion precursor was carefully added to the extruder by hand and in small portions. Upon sufficient filling and after the required amount of time, the material reached the softening point and was capable of being deformed plastically. The complex exited the extruder through a 0.6 mm wide die plate as a continuous strand and was captured on a Teflon film coated surface, where it was cooled down to room temperature to afford the final extrudate.

### Example 10: Extrusion of a 1:2 complex of minocycline (free base) and magnesium stearate together with Resomer 502

### a) Complex Preparation

The components for the complex formation were weighed and treated as described above under Example 1. Subsequently, the complex solution was spread onto multiple Petri dishes coated with Teflon film, and was covered with perforated aluminum foil. The Petri dishes were dried in a vacuum dying oven at room temperature over at least 8 hours (or overnight). A yellow powder was obtained, which was then removed from the Teflon film by means of a card sheet.

### b) Extrusion Precursor Preparation

Then, comminution and homogenization was performed by means of a Retsch CryoMill. The complex powder (412.3 mg, corresponding to 115 mg of pure minocycline) was weighed in the cryomill chamber together with 587.7 mg of the desired biodegradable polymer (Resomer 502) and two milling balls of 1 cm size in the cryomill jar. The jar was fitted in the cryomill and the following parameters were adjusted: upon automatic precooling phase, 6 milling cycles at a frequency of 30 Hz for 150 s each were performed; between the individual milling cycles, intermediate cooling (30 s) was performed at a frequency of 5 Hz. Upon thawing up to room temperature, the extrusion precursor was obtained (1 g, containing 11.5 wt.% pure minocycline).

### c) Extrusion

For extrusion, the three heating zones of a twin-screw extruder were pre-heated at H1 = 49 °C, H2 = 49 °C and H3 = 53 °C. The control unit of the twin-screw extruder was set to 800 rpm, which corresponds to an actual rotation frequency of estimated 150 turns of the screws per minute. The extrusion precursor was carefully added by hand in small portions to the extruded. Upon sufficient filling and after the required amount of time, the material reached the glass transition temperature and was capable of being deformed plastically. The mixture exited the extruder through a 0.6 mm wide die plate as a continuous strand and was captured on a Teflon film coated surface, where it was cooled down to room temperature to afford the final extrudate. The obtained extrudate is shown in Fig. 9 (top).

### Example 11: Extrusion of a 1:2 complex of minocycline (free base) and magnesium stearate together with Resomer 503

An extrudate containing the 1:2 complex of minocycline (free base) and magnesium stearate from Example 1 together with Resomer 503 was prepared in analogous manner as described in Example 10, wherein Resomer 503 was used instead of Resomer 502. The obtained extrudate is shown in Fig. 9 (bottom).

### Example 12: Extrusion of a 1:2 complex of minocycline (free base) and magnesium stearate together with Resomer 502/503 (1:1) and 10% glycerol monostearate (GMS)

An extrudate was prepared in analogous manner as described in Example 10, wherein additionally glycerol monostearate (10 wt.% based on the total weight of the mixture) were used, and a mixture of Resomer 502 and Resomer 503 (1:1 w/w) in total amount adding up to 100 wt.% was used instead of Resomer 502.

### Example 13: Extrusion of a 1:2 complex of minocycline (free base) and magnesium stearate together with PEG-PLGA (Expansorb DLG 50-6P) and 10% PEG 1500.

The complex powder prepared as described above under Examples 1 and 9 (412.3 mg, corresponding to 115 mg of pure minocycline and 297.3 mg magnesium stearate) was weighed in a Retsch CryoMill chamber together with the desired biodegradable polymer (Expansorb DLG 50-6P, 487.7 mg), plasticizer (PEG 1500, 100 mg) and two milling balls of 1 cm size. Then, comminution and homogenization was performed. The container was fitted in the cryomill and the following parameters were adjusted: upon automatic precooling phase, 5 milling cycles at a frequency of 30 Hz for 150 s each were performed; between the individual milling cycles, intermediate cooling (30 s) was performed at a frequency of 5 Hz. Upon thawing up to room temperature, the extrusion precursor was obtained (1 g, containing 11.5 wt.% pure minocycline).

Extrusion was performed in an analogous manner as described in Example 10.

### Example 14: Release properties of the extrudates

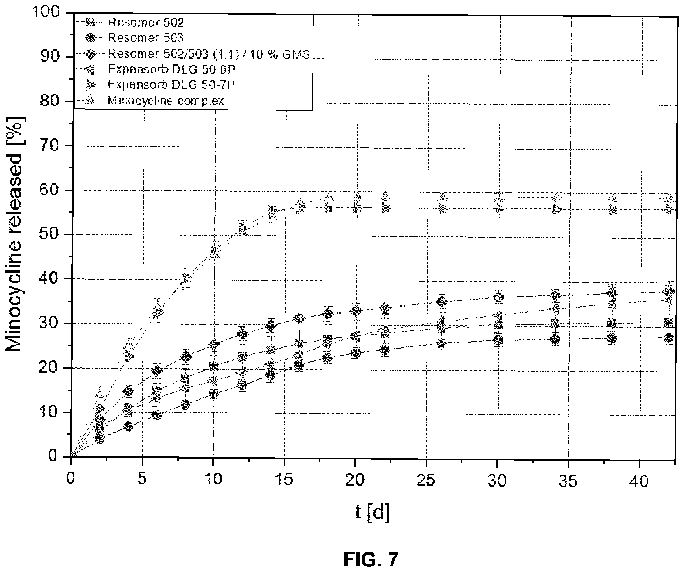

Fig. 7 clearly shows the contribution of the complexes according to the invention to the delayed release of minocycline. Extrudates obtained by melt extrusion as described in Examples 9-13 were cut into 4 mm long pieces and incubated in 1 mL of buffer at pH 7.0 and 37°C. The extrudates of Examples 11-13 have a minocycline content of 11.5%; the pure complex extrudate of Example 9 has a minocycline content of 27.89%. Each time a sample was drawn from the incubation solutions, the entire buffer was exchanged and the minocycline content in the old buffer was determined by means of HPLC. As can be seen from Fig. 7, the pure complex extrudate of Example 9 shows a delayed release of up to almost three weeks. At the end of the experiment, the amount of minocycline remaining residuals in the extrudate of Example 9 was analyzed and it was shown that no active substance was present any more. It can be considered that the remaining 40% have decomposed over the time.

As can be seen from the curves for Examples 10-12, the addition of PLGA polymers leads to further release delay. In this case, about 10-13% of remaining minocycline were still present at the end of the experiment. This may be partially attributed to the acidic microenvironment in PLGA polymers and the slightly better stability in acidic medium, as demonstrated in Fig. 4.

Furthermore, the addition of an excipient such as glycerol mono stearate (Example 12) can accelerate the release if desired.

Finally, the PEG-PLGA-based extrudate of Example 13 containing Expansorb DLG50-6P and 10% of PEG 1500 as a plasticizer also shows advantageous sustained release properties. Additionally, this extrudate has an optimized flexibility and mechanical formability.

### Example 15: Further properties of the extrudates

### a) Mechanical Properties

Fig. 8 shows force-displacement diagrams upon deformation of extrudates fitted in a knife edge blade geometry. For these experiments, the extrudates were installed on a slide on a Brookfield CT3 texture analyzer equipped with a knife edge blade unit and the necessary weight force required for a predetermined penetration depth of 0.5 mm at a velocity of 0.02 mm/sec. was determined. The upper diagram shows brittle properties or extrudates containing Resomer 502, 503 and their mixture with and without glycerol monostearate. PEG-PLGA extrudates with different Expansorbs (lower diagram) show an almost linear relationship between penetration depth and necessary force. The absence of a force drops indicates an irreversible deformation which can be considered to be plastic. Macroscopically, the extrudates exhibit excellent flexibility. The composition with 10% PEG 1500 (Example 13) exhibits no change with respect to the mechanical properties even upon prolonged storage in a refrigerator. In contrast, the composition with 5% PEG hardens partially during storage and behaves rather like the corresponding extrudate without addition of a PEG.

### b) Mucoadhesive Properties

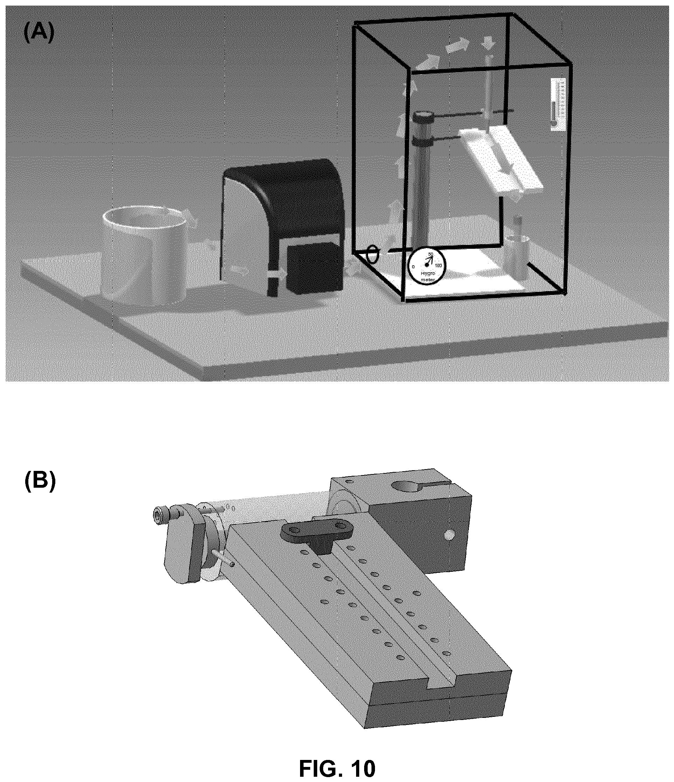

The retention ability of extrudate preparations was tested in a retention analysis experimental setup as shown in Fig. 10A. Porcine intestine mucosa obtained from Tönnies Lebensmittel GmbH & Co. KG (washed, portioned, packaged and frozen or used immediately) was used as a model surface fixed in the channel shown in Fig. 10B. Upon application of a continuous flow of deionized water at a predetermined washing rate (preferably, 300 µL/min) and for a predetermined time, the fraction of the tested preparation still retained by the test surface (mucosa) was determined in a manner similar to the method proposed by Batchelor et al. (Int. J. Pharm. 2002, May 15;238(1-2):123-32). Preparations according to the present invention were found to exhibit good bioadhesion

### Release Kinetics and Antimicrobial Activity

### Example 16: Release Kinetics and Antimicrobial Activity (Minocycline Preparations)

In order to assess the release kinetics and biological activity of the active substances, an approach was chosen taking into consideration the conditions in the oral cavity, namely the flow rate (turnover) and composition of the sulcus in the gingival crevicular fluid (as described in Goodson, JM (2003), Periodontol 2000 31:43-54; Tew, JG et al. (1985), Infect Immun 49:487-93).

### a) Release Kinetics

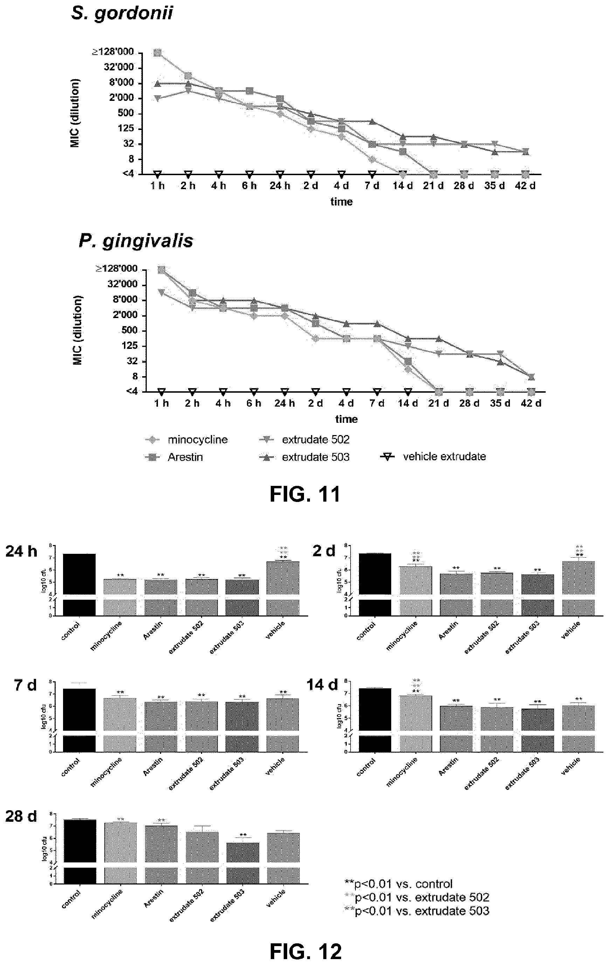

Specifically, samples of the following substances were tested, each containing an amount of active substance equivalent to 1 mg minocycline (except the vehicle extrudate):
- minocycline hydrochloride powder (purchased from SIGMA, Product No. M9511-25MG; in Fig. 11/12: "minocycline")
- minocycline/PLGA microspheres (Arestin® from Orapharma Inc. Europe, PZN 1295115; 1 capsule = 1 mg minocycline; in Fig. 11/12: "Arestin")
- Example 11 (in Fig. 11/12: "extrudate 503")
- Example 10 (in Fig. 11/12: "extrudate 502")
- control consisting of biodegradable polymer without tetracycline compound (in Fig. 11/12: "vehicle extrudate").

Samples were placed in dark tubes and an appropriate amount of buffered saline containing serum albumin (phosphate buffered saline with 15 mg/mL BSA) was added. At given time intervals, the samples were centrifuged, a predetermined amount of the buffer was removed and stored (eluate), and a fresh portion of buffer was then added. Eluates were collected over the course of seven weeks, as shown in Table 4, and tested with respect to their antimicrobial activity using the methods described below (minimal inhibitory concentration determination and biofilm formation inhibition).

**Table 4. Pipetting chart for eluate samples drawn for release kinetics evaluation: * denotes eluates removed at these times were used in the MIC determination; ^{#}denotes eluates removed at these times were used in the biofilm formation inhibition.**

| Time | Removal (µL) | Addition (µL) | Time | Removal (µL) | Addition (µL) |
|---|---|---|---|---|---|
| 0 | 23.5 | | 14 d*^{#} | 3'500 | 3'304 |
| 30 min | 22 | 22 | 17.5 d | 3'304 | 3'304 |
| 60 min* | 22 | 44 | 21 d* | 3'304 | 3'108 |
| 2 h* | 44 | 44 | 24.5 d | 3'108 | 3'108 |
| 3 h | 44 | 44 | 28 d*^{#} | 3'108 | 2'912 |
| 4 h* | 44 | 88 | 31.5 d | 2'912 | 2'912 |
| 6 h* | 44 | 792 | 35 d* | 2'912 | 2'716 |
| 24 h* ^{#} | 792 | 1'056 | 38.5 d | 2'716 | 2'716 |
| 2 d* ^{#} | 1'056 | 1'056 | 42 d* | 2'716 | 2'520 |
| 3 d | 1'056 | 1'056 | 45.5 d | 2'520 | 2'520 |
| 4 d* | 1'056 | 1'056×3=3'168 | 49 d* | 2'520 | 2'324 |
| 7 d* ^{#} | 3'168 | 3'500 | 52.5 d | 2'324 | 2'324 |
| 10,5 d | 3'500 | 3'500 | 56 d* | 2'324 | |

**Table 5. Effective concentration of minocycline versus Streptococcus gordonii ATCC 10558 and Porphyromonas gingivalis ATCC 33277 up to 42 d, calculated based on the MIC of the eluates (dilutions)**

| | **Minocycline** | | **Arestin** | | **Extrudate 502** | | **Extrudate 503** | |
|---|---|---|---|---|---|---|---|---|
| | ***S. gord.*** | ***P. ging.*** | ***S. gord.*** | ***P. ging.*** | ***S. gord.*** | ***P. ging.*** | ***S. gord.*** | ***P. ging.*** |
| **1 h** | >16000 | >32000 | >16000 | >32000 | 1000 | 4000 | 4000 | >32000 |
| **2 h** | 8000 | 2000 | 8000 | 4000 | 2000 | 1000 | 4000 | 2000 |
| **4 h** | 2000 | 1000 | 2000 | 1000 | 1000 | 1000 | 2000 | 2000 |
| **24 h** | 250 | 500 | 1000 | 1000 | 500 | 1000 | 500 | 1000 |
| **2 d** | 62.5 | 62.5 | 125 | 250 | 125 | 250 | 250 | 500 |
| **7 d** | 4 | 62.5 | 15.6 | 62.5 | 15.6 | 62.5 | 125 | 250 |
| **14 d** | <2 | 4 | 8 | 8 | 15.6 | 31.3 | 31.3 | 62.5 |
| **21 d** | <2 | <1 | <2 | <1 | 15.6 | 15.6 | 31.3 | 62.5 |
| **28 d** | <2 | <1 | <2 | <1 | 15.6 | 15.6 | 15.6 | 15.6 |
| **35 d** | <2 | <1 | <2 | <1 | 15.6 | 15.6 | 8 | 8 |
| **42 d** | <2 | <1 | <2 | <1 | 8 | 2 | 8 | 2 |

### b) Minimal Inhibitory Concentration (MIC) Determination

The dilutions were determined at which growth of two indicator strains (*Porphyromonas gingivalis* ATCC 33277, *Streptococcus gordonii* ATCC 10558) was still inhibited (minimal inhibitory concentration, MIC). For this purpose, samples the eluates were mixed with nutrition medium and the respective bacterial strain and incubated over 18 h at 37 °C under aerobic conditions. The approach and the media used were in accordance with the recommendations of The European Committee on Antimicrobial Susceptibility Testing - EUCAST. Each determination was performed double; growth control was performed each time in one well, respectively.

Fig. 11 shows at which dilution degree growth inhibition can still be observed (i.e. the minimal inhibitory concentrations of the eluates (dilution) versus S. *gordonii* ATCC 10558 and *P. gingivalis* ATCC 33277 up to 42 d). An activity of pure minocycline and the commercial product (Arestin) was present up to 21 d, whereas extrudate preparations 502 and 503 showed activity until the end of the observation (42 d).

In Table 5, based on the MIC for minocycline against the two strains (*P. gingivalis* ATCC 33277: 0.25 µg/mL, S. *gordonii* ATCC 10558: 0.5 µg/mL), the effective concentration of the active substance was calculated. These data also highlight the activity of the eluates over a prolonged period of time.

### c) Biofilm Formation Inhibition

The surface of a 96-well plate was coated with 10 µL of eluate. Upon addition of a 6-species mixture of periodontitis-associated bacteria, a biofilm was formed over 6 h (as described in, e.g., Jurczyk K., Nietzsche S., Ender C., Sculean A., Eick S. In-vitro activity of sodium-hypochlorite gel on bacteria associated with periodontitis. Clin Oral Investig. 2016 Nov;20(8):2165-2173). Then, the number of still vital bacteria in the biofilm (colony forming units = cfu) was determined.

Fig. 12 shows the inhibitory activity of eluates obtained after 24 h, 2 d, 7 d, 14 d und 28 d against biofilm formation. Biofilm formation inhibition can be attributed to antimicrobial activity, on the one hand, and to interference with steps of biofilm formation (attachment of microorganisms to surfaces or to already attached microorganisms (co-aggregation), matrix formation), on the other hand. Here, the eluate from the vehicle seems to show some anti-biofilm properties as well. After 24 h of incubation, no difference was present between the tested substances. Later on, however, all of the preparations according to the present invention show better activity than pure minocycline. After 28 days, a significant inhibition with Extrudate 503 (-1.9 log₁₀ cfu) was still present.

### Example 17: Release Kinetics and Antimicrobial Activity (Doxvcvcline Preparations)

In order to assess the release and kinetics and the biological activity of the doxycycline preparations, an analogous approach was chosen. Specifically, samples of the following substances were tested, each containing an amount of active substance equivalent to 1 mg doxycycline (except the vehicle extrudate):
- doxycycline hyclate (1.15 mg doxycycline hyclate = 1 mg doxycycline)
- doxycycline gel (Ligosan®, 1 cartridge = 260 mg, 14% doxycycline)
- doxycycline extrudate (corresponding to 11,5% doxycycline, prepared in an analogous manner as described in Example 13)
- vehicle extrudate.

The tests were performed in an analogous manner as described in Example 16 above. The release kinetics and antimicrobial activity of the preparation according to the present invention relative to the reference samples were found to be comparable with the results obtained for minocycline preparations.

## Claims

1. A complex comprising a tetracycline compound (TC) or a pharmaceutically acceptable salt, hydrate or solvate thereof and a divalent metal carboxylate (MA₂), wherein:
the molar ratio TC : MA₂ is in the range of 1 : 0.8-3.0;
the divalent metal cation M is an earth alkaline metal cation; and
A is a carboxylate anion derived from a C₈-C₂₄ carboxylic acid.

2. The complex according to claim 1, wherein the tetracycline compound (TC) is selected from minocycline, doxycycline, tetracycline, chlortetracycline, oxytetracycline, rolitetracycline, tigecycline, demeclocycline, lymecycline, meclocycline, methacycline, omadacycline, sarecycline, eravacycline, clomocycline, 9-(N,N-dimethylglycylamido)-6-demethyl-6-deoxytetracycline and 9-(N,N-dimethylglycylamido)-minocycline.

3. The complex according to any one of the preceding claims, wherein:
the ratio TC: MA₂ is 1:2; and/or
M is selected from Mg²⁺ and Ca²⁺; and/or
A is a carboxylate anion derived from a carboxylic acid selected from arachidic acid (C₂₀), stearic acid (C₁₈), palmitic acid (C₁₆), myristic acid (C₁₄), lauric acid (C₁₂), capric acid (C₁₀), caprylic acid (C₈), hydroxystearic acid, oleic acid, linoleic acid, linolenic acid and ricinoleic acid.

4. The complex according to any one of the preceding claims having the formula [(TC)·2(MgA₂)], wherein the tetracycline compound (TC) is minocycline or a pharmaceutically acceptable salt, hydrate or solvate thereof.

5. A pharmaceutical preparation comprising the complex according to any one of claims 1-4 and optionally one or more pharmaceutically acceptable excipient(s).

6. The pharmaceutical preparation according to claim 5, which is arranged for topical administration and/or is selected from the group consisting of extrudate, particle, granule, powder, film, strip, compact, chip, paste, cream, gel, emulsion, suspension, liniment, ointment, balm, lotion, eye drops, spray, topical aerosol, topical solution, topical suspension, skin patch and nonwoven.

7. The pharmaceutical preparation according to claim 5 or 6 further comprising, as an excipient, one or more biodegradable polymer(s) selected from biodegradable polyesters, such as poly(glycolic acid) (PGA), poly(lactic acid) (PLA), poly(lactic-co-glycolic acid) (PLGA), polyhydroxybutyric acid (PHB) or polycaprolactone (PCL); mixed biodegradable polyesters, such as PLA-PCL and PLGA-PCL; biodegradable PEGylated diblock (AB) or triblock (ABA or BAB) copolymers, such as PEG-PLA, PEG-PLGA, PEG-PCL and PEG-PCL-PLGA; and pectins, and/or
wherein the total content of the tetracycline compound (TC) is in the range of 5-20 wt.%.

8. The pharmaceutical preparation according to any one of claims 5-7, which in the form of a strand-shaped extrudate, preferably having an essentially circular or an essentially elliptical cross section and/or a maximum cross-sectional diameter in the range of 0.1-10 mm.

9. A method for manufacturing the complex according to any one of claims 1-4, the method comprising the following steps:
(a) mixing a tetracycline compound (TC) or a pharmaceutically acceptable salt, hydrate or solvate thereof and a divalent metal carboxylate (MA₂) in a molar ratio of 1 : 0.8-3.0 with an organic solvent to obtain a dispersion, wherein the divalent metal cation M is an earth alkaline metal cation and A is a carboxylate anion derived from a C₈-C₂₄ carboxylic acid;
(b) heating said dispersion to form the complex;
(c) removing the solvent to obtain the complex.

10. A method for manufacturing the pharmaceutical preparation according to claim 8, the method comprising the following steps:
(d) comminuting the complex according to any one of claims 1-4 and, if present, one or more pharmaceutically acceptable excipients to obtain an extrusion precursor;
(e) extruding said extrusion precursor at a temperature above room temperature;
(f) cooling the product of step (e) to obtain the pharmaceutical preparation in the form of a strand-shaped extrudate.

11. The complex according to any one of claims 1-4 or the pharmaceutical preparation according to any one of claims 5-8 for use in a method for treatment of the human or animal body.

12. The complex according to any one of claims 1-4 or the pharmaceutical preparation according to any one of claims 5-8 for use in a method for therapy and/or prophylaxis of (i) a bacterial infection; or (ii) a bacterial infection caused by one or more bacteria selected from the group consisting of *Porphyromonas gingivalis, Prevotella intermedia, Tannerella forsythia, Streptococcus gordonii, Fusobacterium nucleatum, Actinomyces naeslundii* and *Parvimonas micra.*

13. The complex according to any one of claims 1-4 or the pharmaceutical preparation according to any one of claims 5-8 for the use according to claim 12, wherein antibiotic activity is maintained over a period of at least 21 days.

14. The complex according to any one of claims 1-4 or the pharmaceutical preparation according to any one of claims 5-8 for use in a method for therapy and/or prophylaxis of an acute, chronic or recurrent periodontal disease.

15. The complex according to any one of claims 1-4 or the pharmaceutical preparation according to any one of claims 5-8 for use in the method according to claim 14, wherein the periodontal disease is selected from dental plaque-induced gingival diseases, periodontitis, chronic periodontitis, aggressive periodontitis, periodontitis as a manifestation of systemic diseases, necrotizing periodontal diseases, abscesses of the periodontium, periodontitis associated with endodontic lesions, peri-implant mucositis, peri-implantitis and endodontic infections.
